# EUROPEAN PATENT APPLICATION

(11) **EP 3 616 733 A1**
(43) Date of publication of application: **04.03.2020**
(21) Application number: 18791388.4
(22) Date of filing: 27.04.2018
(51) Int. Cl.: A61M 1/36, C12M 1/12, C12M 3/00, C12N 5/0735, C12N 15/00, C12Q 1/68

(54) **EXTRACORPOREAL ARTIFICIAL LIVER, DEVICE FOR EXTRACORPOREAL ARTIFICIAL LIVER OR HEPATOCYTE CULTURE**

(30) Priority: 28.04.2017 JP 2017090335
(71) Applicant: Yonemitsu, Yoshikazu, Fukuoka-shi, Fukuoka 810-0023 (JP); GAIA BioMedicine Inc., Chuo-ku Fukuoka-shi Fukuoka 810-0023 (JP)
(72) Inventor: YONEMITSU Yoshikazu, Fukuoka-shi Fukuoka 810-0023 (JP); HARADA Yui, Fukuoka-shi Fukuoka 8190395 (JP)
(74) Representative: Vossius & Partner Patentanwälte Rechtsanwälte mbB
(86) International application number: PCT/JP2018/017122
(87) International publication number: WO 2018/199274

(57) **Abstract**

An object of the present invention is to provide an artificial liver using highly functional hepatocytes. There is provided an extracorporeal artificial liver having at least one module comprising the followings: a plasma ingredient inlet port, and a plasma ingredient outlet port; a plasma ingredient circulation chamber having the plasma ingredient inlet port, and the plasma ingredient outlet port; and a hepatocyte culture chamber provided adjacently to the plasma ingredient circulation chamber and separated from the plasma ingredient circulation chamber with a separation membrane through which ammonia can permeate, but hepatocytes cannot permeate, in which hepatocytes having an ammonia-metabolizing ability are cultured.

SEQUENCE LISTING

<110>  YONEMITSU, Yoshikazu
       GAIA BioMedicine Inc.

<120>  Extracorporeal artificial liver and device for extracorporeal
artificial liver or culture of hepatocytes

<130>  AC3203 EP S3

<140>  EP 18 79 1388.4
<141>  2018-04-27

<150>  JP 2017-090335
<151>  2017-04-28

<160>  10

<170>  PatentIn version 3.5

<210>  1
<211>  26
<212>  DNA
<213>  Artificial Sequence

<220>
<223>  PCR primer, hALB For

<400>  1
cctttggcac aatgaagtgg gtaacc                                          26


<210>  2
<211>  25
<212>  DNA
<213>  Artificial Sequence

<220>
<223>  PCR primer, hALB Rev

<400>  2
cagcagtcag ccatttcacc atagg                                           25


<210>  3
<211>  27
<212>  DNA
<213>  Artificial Sequence

<220>
<223>  PCR primer, hHNF4a For

<400>  3
ctgctcggag ccaccaagag atccatg                                         27


<210>  4
<211>  25
<212>  DNA
<213>  Artificial Sequence

<220>
<223>  PCR primer, hHNF4a Rev

<400>  4
atcatctgcc acgtgatgct ctgca                                          25


<210>  5
<211>  25
<212>  DNA
<213>  Artificial Sequence

<220>
<223>  PCR primer, hOCT4 For

<400>  5
gacaacaatg aaaatcttca ggaga                                          25


<210>  6
<211>  23
<212>  DNA
<213>  Artificial Sequence

<220>
<223>  PCR primer, hOCT4 Rev

<400>  6
ttctggcgcc ggttacagaa cca                                            23


<210>  7
<211>  22
<212>  DNA
<213>  Artificial Sequence

<220>
<223>  PCR primer, hCYP3A4 For

<400>  7
ccttacatat acacaccctt tg                                             22


<210>  8
<211>  23
<212>  DNA
<213>  Artificial Sequence

<220>
<223>  PCR primer, hCYP3A4 Rev

<400>  8
ggttgaagaa gtcctcctaa gct                                            23


<210>  9
<211>  19
<212>  DNA
<213>  Artificial Sequence

<220>
<223>  PCr primer, h\203À-actin For

<400>  9
tcaccaccac ggccgagcg                                                 19

<210>  10
<211>  20
<212>  DNA
<213>  Artificial Sequence

<220>
<223>  PCR primer, h\203À-actin Rev

<400>  10
tctccttctg catcctgtcg                                                    20

## Description

### Technical Field

The present invention relates to an extracorporeal artificial liver using hepatocytes having ammonia-metabolizing ability and a device for extracorporeal artificial liver or culture of hepatocytes.

### Background Art

Even in these days enjoying advanced medical techniques including those for acute stage management, there are still never-ceasing deaths from liver failure in Japan. In particular, mortality rate of fulminant hepatitis is as extremely high as 70 to 90%, and it is a disease of bad prognosis. Although liver transplantation provides the highest curative effect as the treatment of liver failure, there are many cases where liver transplantation cannot be carried out, because of shortage of donors or drastic progress of acute hepatic failure.

A hybrid artificial liver support system (HALSS) using hepatocytes cultured in an artificial structure for assisting the liver functions is expected a system to be used for assisting liver functions for the period until liver transplantation is carried out, or patient's own liver regenerates.

As the hybrid artificial liver support system, use of a culture vessel of hollow fiber type, multi-layer plate type or the like that can maintain cells at a high density has conventionally been examined. In order to supply sufficient oxygen to hepatocytes contained in such a culture vessel at a high density, use of an oxygenation device has been examined. For example, Patent document 1 proposes a system for perfusion and culture in a hollow fiber type artificial liver, which comprises a perfusion circuit consisting of a hollow fiber module enclosing hepatocytes in internal cavities of hollow fibers, an oxygenation device, a liquid feeding pump, a liquid reservoir, and pipes connecting them, and wherein a circulation circuit is formed so that the perfusate is flown outside the hollow fiber module from the liquid reservoir, and returned to the liquid reservoir again by the liquid feeding pump. Patent document 2 proposes a hybrid artificial liver comprising a blood distribution channel having a plasma separation device and a hemocyte-plasma mixing part in this order between a blood inlet and a blood outlet, and a plasma circulation channel that flows plasma separated by the plasma separation device into the hemocyte-plasma mixing part via a plasma treatment device containing hepatocytes, wherein an oxygenation device is provided in the plasma treatment device or in the upstream thereof for maintaining dissolved oxygen concentration in the plasma of the outlet side of the plasma treatment device to be 0.5 ppm or higher.

As for hybrid artificial livers, cells to be used and device configuration for using such cells have also been examined. For example, Patent document 3 proposes, as a cell strain suitable for use in a liver function-assisting device such as hybrid artificial liver, a human liver-derived cell strain transformed with a gene for a drug-metabolizing enzyme such as P450 3A4 and an ammonia-metabolizing enzyme gene such as a glutamine synthetase gene. Patent document 4 proposes, as a blood purifying apparatus that can reduce extracorporeally circulating blood volume and can enhance purification efficiency, a blood purifying apparatus comprising a living body-side blood circulating circuit having a plasma separator for separating blood into hemocytes and plasma, and an artificial organ module-side plasma circulation circuit having an artificial organ module for purifying plasma separated by the plasma separator, wherein the artificial organ module-side plasma circulation circuit forms a closed circuit independent from the living body-side blood circulation circuit.

### Prior art references

### Patent documents

Patent document 1: Japanese Patent Unexamined Publication (KOKAI) No. 10-33671
Patent document 2: Japanese Patent Unexamined Publication (KOKAI) No. 10-234850
Patent document 3: Japanese Patent Unexamined Publication (KOKAI) No. 2003-274963
Patent document 4: Japanese Patent Unexamined Publication (KOKAI) No. 2004-49301

### Disclosure of the Invention

### Object to be Achieved by the Invention

The inventors of the present invention examined artificial hepatocytes used for hybrid artificial livers, and obtained a hepatocyte that can theoretically infinitely proliferate, and has high ammonia-metabolizing ability (Japanese Patent Application No. 2016-037518, which is not published at the time of filing of the present application). Then, since a culture of this hepatocyte can form a reticular structure in the form of a sheet, they conducted various researches on the shape of a module that can constitute an extracorporeal artificial liver in which such a reticular structure in the form of a sheet is maintained. As a result, they thought of a culture vessel constituted by two chambers of a part in which plasma ingredients from a patient circulate, and a part for culturing hepatocytes separated by a separation membrane from the former part, found that such a culture vessel is suitable for culturing various hepatocytes, and accomplished the present invention.

The present invention thus provides the followings.
[1] An extracorporeal artificial liver having at least one module comprising the followings:
   - a plasma ingredient inlet port, and a plasma ingredient outlet port;
   - a plasma ingredient circulation chamber having the plasma ingredient inlet port, and the plasma ingredient outlet port; and
   - a hepatocyte culture chamber provided adjacently to the plasma ingredient circulation chamber and separated from the plasma ingredient circulation chamber with a separation membrane through which ammonia can permeate, but hepatocytes cannot permeate, in which hepatocytes having an ammonia-metabolizing ability are cultured.
[2] The artificial liver according to 1, wherein the plasma ingredient circulation chamber is disposed above the hepatocyte culture chamber.
[3] The artificial liver according to 1 or 2, wherein the hepatocytes are artificial hepatocytes that have an ammonia-metabolizing ability of 100 µg/dl/24h or higher, and can constitute a reticular structure.
[4] The artificial liver according to any one of 1 to 3, wherein the hepatocytes are cultured under a serum-free and feeder-free environment.
[5] The artificial liver according to any one of 1 to 4, wherein at least 1.0 x 10⁶ of hepatocytes are cultured per one module.
[6] The artificial liver according to any one of 1 to 5, wherein the artificial liver comprises a plurality of modules, and 5.0 x 10⁹ to 5.0 x 10¹¹ of hepatocytes are cultured.
[7] The artificial liver according to any one of 1 to 6, which has an effective culture area of 1 x 10⁴ to 1 x 10⁶ cm².
[8] A device for an extracorporeal artificial liver or for culturing hepatocytes, which comprises at least the followings:
   - a plasma ingredient inlet port, and a plasma ingredient outlet port;
   - a plasma ingredient circulation chamber having the plasma ingredient inlet port, and the plasma ingredient outlet port; and
   - a hepatocyte culture chamber for culturing hepatocytes provided adjacently to the plasma ingredient circulation chamber and separated from the plasma ingredient circulation chamber with a separation membrane through which ammonia can permeate, but hepatocytes and cannot permeate.
[9] The device according to 8, wherein the hepatocyte culture chamber has a cell introduction port for introducing hepatocytes.
[10] The device according to 9, wherein the hepatocyte culture chamber has an air-discharging port for discharging air.
[11] The device according to any one of 8 to 10, wherein a means for suppressing deformation of the separation membrane is provided in the hepatocyte culture chamber.

The present invention also provides the followings.
[1] An artificial hepatocyte that has an ammonia-metabolizing ability of 100 µg/dl/24h or higher, and can constitute a reticular structure.
[2] The artificial hepatocyte according to 1, which can be cultured under a serum-free and feeder-free environment.
[3] The artificial hepatocyte according to 1 or 2, which is induced from an artificial pluripotent stem (iPS) cell.
[4] The artificial hepatocyte according to 3, wherein the iPS cell is produced by using a Sendai virus vector.
[5] A method for preparing an artificial hepatocyte comprising the following steps:
   the differentiation induction step 1 of culturing an iPS cell in a differentiation induction medium I containing activin A,
   the differentiation induction step 2 of culturing a cell obtained in the differentiation induction step 1 in a differentiation induction medium II containing bone morphogenetic protein 4 (BMP4) and fibroblast growth factor 2 (FGF2); and
   the differentiation induction step 3 of culturing a cell obtained in the differentiation induction step 2 in a differentiation induction medium III containing hepatocyte growth factor (HGF), oncostatin M, dexamethasone, and N,N'-(methylenebis)(4,1-phenylene)diacetamide (FH1) and/or 2-([N-(5-chloro-2-methylphenyl)(methylsulfonamido)-N-(2,6-difluorophenyl)acetamide (FPH1) to obtain an artificial hepatocyte.
[6] The preparation method according to 5, wherein the iPS cell has been produced by using a Sendai virus vector.
[7] The preparation method according to 5 or 6, wherein the differentiation induction medium III contains FH1 and FPH1.
[8] The preparation method according to any one of 5 to 7, wherein the differentiation induction steps 1 to 3 are performed in a serum-free and feeder-free environment.
[9] The preparation method according to any one of 5 to 8, wherein the differentiation induction step 1 and the differentiation induction step 2 are performed for 2 to 14 days in total, and the differentiation induction step 3 is performed for 2 to 14 days.

### Effect of the Invention

The present invention provides a hybrid artificial liver comprising at least one module that maintains hepatocytes in the form of a sheet.

The present invention provides a device for a hybrid artificial liver, and a device for culturing hepatocytes, which can maintain hepatocytes as a tissue in the form of a sheet, and in which a circulating flow is not directly transmitted to the hepatocytes.

### Brief Description of the Drawings

[Fig. 1] Fig. 1 is a sectional view of an example of an embodiment of the module that constitutes the artificial liver.
[Fig. 2] Fig. 2 is a schematic view of an example of an embodiment of the system using the artificial liver. The arrows in the drawing represent directions of flows of blood and so forth in the circuits.
[Fig. 3] iPS cells (CiPS) derived from cord blood CD34 positive-cells.
[Fig. 4] iPS cells (CiPS) derived from cord blood CD34 positive-cells.
[Fig. 5] Confirmation of elimination of rSeV vector from CiPS.
[Fig. 6] Highly functional hepatocytes induced from optimized iPS cells.
[Fig. 7] Confirmation of the amount of metabolized ammonia. It was found that use of the compounds (FH1, FPH1) provides higher metabolism-enhancing effect compared with use of the feeder cells (HUVECs, MSCs).
[Fig. 8] Confirmation of the amount of metabolized ammonia. It was found that that the obtained iPS-HEP cells can have an ammonia-metabolizing ability comparable to the metabolism ability of the primary hepatocytes (160 to 200 µg/dl/24h) depending on the culture conditions.
[Fig. 9] Confirmation of a hepatocyte marker (RT-PCR).
[Fig. 10] Confirmation of a hepatocyte marker (RT-PCR).
[Fig. 11] Confirmation of a hepatocyte marker (RT-PCR).

### Modes for Carrying out the Invention

The present invention provides an extracorporeal artificial liver having at least one module comprising the followings:
- a plasma ingredient inlet port, and a plasma ingredient outlet port;
- a plasma ingredient circulation chamber having the plasma ingredient inlet port, and the plasma ingredient outlet port; and
- a hepatocyte culture chamber provided adjacently to the plasma ingredient circulation chamber and separated from the plasma ingredient circulation chamber with a separation membrane through which ammonia can permeate, but hepatocytes cannot permeate, in which hepatocytes having an ammonia-metabolizing ability are cultured.

### <Module, artificial liver, and culture vessel for hepatocytes>

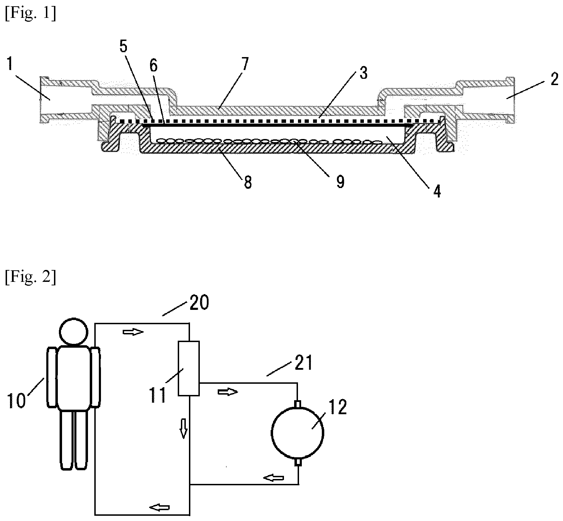

Fig. 1 is a sectional view of an example of an embodiment of the module that constitutes the artificial liver. The inside of the module is divided into a plasma ingredient circulation chamber 3 and a hepatocyte culture chamber 4 with a separation membrane 5. The module can be formed by putting the separation membrane 5 between a module lid 7 and a module body 8. Although the plasma ingredient circulation chamber 3 and the hepatocyte culture chamber 4 may be disposed in any manner, it is preferable to dispose the plasma ingredient circulation chamber 3 above the hepatocyte culture chamber 4 as shown in Fig. 1. The plasma ingredient circulation chamber 3 is provided with a plasma ingredient inlet port 1 and a plasma ingredient outlet port 2. Hepatocytes 9 are cultured in the hepatocyte culture chamber 4. The hepatocytes may be cultured under a serum-free and feeder-free environment. Examples of usable hepatocytes will be detailed later. In the module, a means for suppressing deformation of the separation membrane 5, for example, a separation membrane support 6, may be provided so that the separation membrane 5 should not deform. The hepatocyte culture chamber 4 may be provided with a cell introduction port for introducing hepatocytes, and an air discharging port for discharging air at the time of introducing hepatocytes.

At the time of use, the inside of the module is substantially fully filled with plasma ingredients, and plasma ingredients circulate therein via the plasma ingredient inlet port 1 and the plasma ingredient outlet port 2. Comparatively low molecule ingredients that can pass through the separation membrane 5 diffuse from the plasma ingredient circulation chamber 3 into the hepatocyte culture chamber 4.

The plasma ingredients that circulate through the inside of the module are not limited to those of plasma, and they may be those of the whole blood containing plasma. When the module is used as a culture vessel of hepatocytes for a toxicity test or the like as described later, a medium suitable for culture of the cells is circulated in the module as the plasma ingredients. Before introducing hepatocytes, before use as an artificial liver or the like, after such use, and so forth, an isotonic solution or the like may be circulated for the purpose of washing, adjustment of environment, conditioning, or the like. The plasma ingredients referred to in the present invention include plasma, whole blood, medium, and isotonic solution, unless especially indicated.

If there is used the configuration that the plasma ingredient circulation chamber 3 and hepatocyte culture chamber 4 are separated with the separation membrane 5, hepatocytes do not enter into the plasma ingredient circulation chamber. Into the hepatocyte culture chamber, only comparatively low molecule ingredients pass through the separation membrane and diffuse into the hepatocyte culture chamber from the circulating flow, and the circulating flow does not directly flow into the hepatocyte culture chamber. Since the fluid in the hepatocyte culture chamber does not significantly move, damage of the hepatocytes themselves cultured in the hepatocyte culture chamber and damage of a structure formed by the hepatocytes can be prevented. It is thought that the functions of the hepatocytes can be therefore highly maintained. By using such a structure, the module can be made thinner, and can be made to have a shape suitable for stacking it. Therefore, when a large number of hepatocytes are required, area of the separation membrane of a certain level or larger can be secured by using a plurality of modules. Further, sufficient amount of oxygen and so forth can also be supplied for such a large number of hepatocytes, and therefore the wastes such as ammonia can be fully metabolized by the hepatocytes.

The shape of the whole module is not particularly limited, and it may have an arbitrary shape such as a round shape or a square shape. The size of the module is not also particularly limited, and it may have an arbitrary size. According to a particularly preferred embodiment, the module has such a size that 1.0 x 10⁶ of hepatocytes can be cultured per one module. Such a module can have an internal volume of 1 to 10 ml, and an area of surface to which hepatocytes can adhere at the time of the culture (effective culture area) of 1 to 20 cm².

The material that constitutes the module is preferably selected from those suitable for culture of hepatocytes. In particular, for the internal surface of the hepatocyte culture chamber 4, a material that shows compatibility to hepatocyte is preferably selected, because hepatocytes are adhered to it and cultured. The material of the module is preferably a material that can be sterilized, and is preferably transparent. It is also preferably a material acceptable as a material of a medical supply. Specific examples of the material of the module include block copolymers of polysulfone, polypropylene, polyvinyl chloride, polyethylene, polyimide, polycarbonate, polymethylpentene, polystyrene, and so forth.

As the separation membrane, a porous membrane through which ammonia can penetrate, for example, a membrane having a molecule fractionation ability for 300 kDa or larger is used. The separation membrane preferably has such a pore diameter that hepatocytes cannot penetrate, for example, a diameter of 0.65 µm or smaller. As the material of the separation membrane, a material that is acceptable as a material of a medical supply is preferred. A material that can be sterilized is preferred. Specific examples of the material of the separation membrane include cellulose materials and synthetic polymers, more specifically, regenerated cellulose (RC), cuprammonium rayon (CR), saponified cellulose (SCA), surface-modified regenerated cellulose, cellulose acetate (CA), cellulose diacetate (CDA), cellulose triacetate (CTA), polyacrylonitrile (PAN), polymethylmethacrylate (PMMA), ethylene-vinyl alcohol copolymer (EVAL), polysulfone (PS), polyamide (PA), and polyester type polymer alloy (PEPA).

The module may have a means for suppressing deformation of the separation membrane (separation membrane support 6). The means for suppressing deformation of the separation membrane can be provided under the separation membrane, and can be a structure having a lattice or reticular structure and using a material showing no toxicity against hepatocytes or living bodies.

An artificial liver is constituted by at least one of such a module. The number of the module can be determined according to the size of one module, and the number of hepatocytes that should be used for the artificial liver. When, for example, a module having such a size that hepatocytes of a number of 10⁶ order can be cultured (a volume of 1 to 10 ml, and an effective culture area of 1 to 20 cm²) is used, it is expected that 1 to 5 of such modules are used for one mouse having a weight of about 20 g, or 10 to 50 of such modules are used for one rat having a weight of about 200 g.

When the artificial liver is used for a human (adult), it is assumed that 5.0 x 10⁹ to 5.0 x 10¹¹, more precisely, 1 x 10¹⁰ to 1 x 10¹¹, of hepatocytes are required. The effective membrane culture area of such an artificial liver may be 1 x 10⁴ to 1 x 10⁶ cm², more precisely, 1.9 x 10⁴ to 1.9 x 10⁵ cm². When a plurality of modules are used, the modules can be connected in parallel, or can also be connected in series.

In general, hybrid artificial livers are classified into three types, i.e., those extracorporeally installed and connected to a blood vessel, those detained in the inside of the body and connected to a blood vessel, and those detained in the abdominal cavity without being connected with a blood vessel. The artificial liver of the present invention is an extracorporeal artificial liver. An extracorporeal artificial liver is also preferred for obviating risks accompanying cell transfer or the like, when hepatocytes derived from iPS cells are used as described later.

The device comprising the aforementioned module provided by the present invention can be used not only for artificial livers, but also for culture of hepatocytes. Hepatocytes cultured in the device or liver tissues comprising such hepatocytes can be used for various purposes. For example, they can be used for various tests such as safety test; toxicity test; absorption, distribution, metabolism and excretion test (ADME); drug metabolism and pharmacokinetics test (DMPK); drug-drug interaction test; and antiviral activity test. They can also be used for screening of pharmaceuticals (for example, screening for anti-hyperlipidemic drug, therapeutic drug for hypertension, low molecular weight compound drug, or antibody drug), and target screening for drug discovery. They can further be used for disease model; infectious disease model; and substitute for animal model. The device can also be used for biological researches, for example, for the purpose of large scale culture, high density culture, high order culture, and so forth.

When the device is used for culture of hepatocytes, a medium for cell culture can be circulated in the module instead of plasma ingredients.

### <System and circuit>

Fig. 2 is a schematic view of an example the system using the artificial liver according to a certain embodiment. When a plasma separator 11 is used, an extracorporeal circulation system using an artificial liver can be constituted with a first circulation circuit 20 including a patient 10 (patient-side circuit) and a second circulation circuit 21 including an artificial liver 12 (artificial liver-side circuit). Blood extracted from the patient 10 is circulated in the circulation circuit 20 by using a liquid-feeding pump or the like as required. If the blood is introduced into the plasma separator 11, plasma is separated from the blood of the patient, and the separated plasma flows into the circulation circuit 21. In order to flow the plasma into the circulation circuit 21, a liquid-feeding pump may be used. If the plasma is introduced into the artificial liver 12, toxic substances such as ammonia are metabolized by hepatocytes cultured in the module constituting the artificial liver. The plasma introduced into the artificial liver 12 also has a role of supplying required substances such as oxygen to the hepatocytes in the artificial liver. The plasma in which toxic substances are reduced by the hepatocytes is drawn from the artificial liver 12, flown into the circulation circuit 20, combined with the blood flowing in the circulation circuit 20, and returned to the patient 10.

The constitution of the extracorporeal circulation system using the artificial liver of the present invention is not limited to such an embodiment using a plasma separator, and a constitution similar to that of the conventional hemodialysis systems and not using a plasma separator may also be used. Specifically, a constitution consisting of a conventional hemodialysis circuit, but including the artificial liver of the present invention instead of a dialyzer can be used.

### <Hepatocyte>

Hereafter, hepatocytes used for the artificial liver of the present invention will be explained. Various hepatocytes that have been examined for use in the conventional hybrid artificial livers can be used for the present invention.

The hepatocytes may be those of an established cell strain, or primary culture cells of the cells of liver of an adult, fetus, or the like, or may be cells induced from stem cells. Examples of established cell strain include the followings: BRL-3A (rat liver-derived cell strain, producing MSA (somatomedin-like) protein (Buffalo rat)), LMH (chicken hepatocellular carcinoma, diethylnitrosamine-induced), RL-34 (cell strain established from rat normal liver, growth is arrested with collagen (Wistar rat)), ARLJ301-3 (rat liver epithelial cell strain (F344-/DuCrj rat)), FAA-HTC1 (rat liver hepatocellular carcinoma, 2-acetylaminofluorene-induced (F344-/DuCrj rat)), RLN-B2 (cell strain established from rat normal liver (Donryu rat)), RLN-J-5-2 (cell strain established from rat normal liver (Donryu rat)), Ac2F (cell strain established from rat normal liver, karyotype is diploid (Donryu rat)), dRLa-74 Clone 2 (rat liver, adenoma (Donryu rat)), dRLh-84 (rat hepatocellular carcinoma (Donryu rat)), AH66tc (rat ascites hepatoma (Donryu rat)), AH70Btc (rat ascites hepatoma (Donryu rat)), RLN-8 (cell strain established from rat normal liver (Donryu rat)), RLN-10 (cell strain established from rat normal liver (Donryu rat)), Ac2F(DT) (derived from rat Ac2F, 3'-methyl-4-dimethylaminoazobenzene transformed strain (Donryu rat)), Ac2F(ST) (derived from rat Ac2F, spontaneously transformed strain (Donryu rat)), dRLN-4 (rat liver-derived cell strain, 3'-methyl-4-dimethylaminoazobenzene-induced (Donryu rat)), dRLN-9 (rat liver-derived cell strain, 3'-methyl-4-dimethylaminoazobenzene-induced (Donryu rat)), dRLN-6 (rat liver-derived cell strain, 3'-methyl-4-dimethylaminoazobenzene-induced (Donryu rat)), 3'-mRLN-30 (rat liver-derived cell strain, 3'-methyl-4-dimethylaminoazobenzene-induced (Donryu rat)), 3'-mRLN-31 (rat liver-derived cell strain, 3'-methyl-4-dimethylaminoazobenzene-induced (Donryu rat)), 3'-mRLh-2 (rat hepatocellular carcinoma (Donryu rat)), IAR-20 (rat non-transformed hepatocyte strain (BD-IV rat)), JTC-16-P3 (rat ascites hepatoma, derived from JTC-16, strain adjusted to serum-free medium (Donryu rat)), RLC-10.P3 (derived from rat RLC-10 cell, strain adjusted to serum-free medium (JAR-1-rat)), NCTC Clone 1469 (cell strain established from mouse normal liver (C3H/An mouse)), BRL-3A (rat liver-derived cell strain, producing MSA (somatomedin-like) protein (Buffalo rat)), AH601.P3(JTC27) (rat transplantable ascites hepatoma, strain adjusted to serum-free medium (Donryu rat)), AH601(JTC27) (rat transplantable ascites hepatoma (Donryu rat)), M (derived from rat liver, established with DAB (JAR rat)), M.P3 (derived from rat liver, strain adjusted to serum-free and lipid-free medium of M cell strain established with DAB (JAR rat)), NCTC clone 1469 (cell strain established from mouse normal liver (C3H/An mouse)), FLS3 (mouse liver stroma cell strain), FLS5 (mouse liver stroma cell strain), TLR2 (mouse hepatocyte strain, immortalized with the temperature sensitive SV40 large T gene, culture temperature is 33°C (C57BL/6 transgenic mouse)), TLR3 (mouse hepatocyte strain, immortalized with the temperature sensitive SV40 large T gene, culture temperature is 33°C (C57BL/6 transgenic mouse)), WB-F344 (rat liver epithelial cell strain, reported that phenotypes are similar to those of oval cell (Fischer F344 rat)), FF101 (rat hepatocellular carcinoma, 3'-methyl-4-dimethylaminoazobenzene-induced, and serum-free cultured (Donryu rat)), AH601.P3(JTC27) (rat transplantable ascites hepatoma, strain adjusted to serum-free medium), AH-7974.P3 (rat ascites hepatoma, derived from JTC-16, strain adjusted to serum-free medium), RLC-10.P3 (derived from rat RLC-10 cell, strain adjusted to serum-free medium), M.P3 (derived from rat liver, strain adjusted to serum-free and lipid-free medium of M cell strain established with DAB), RI-T (rat liver stellate cell (Ito cell) strain, immortalized with SV40 T antigen (Wistar rat)), TMNK-1 (immortalized human liver endothelial cell), HUH-6 Clone 5 (human hepatoblastoma, well differentiated type), HuH-7 (human hepatocellular carcinoma, differentiated type), HLE (human hepatocellular carcinoma, undifferentiated type), HLF (human hepatocellular carcinoma, undifferentiated type), PLC/PRF/5 (human hepatocellular carcinoma, HBs antigen-positive), HuCCT1 (human bile duct carcinoma), HuH28 (human bile duct carcinoma), JHH-4 (human hepatocellular carcinoma), LI90 (human liver Ito cell, finite proliferation), Alexander cells (human hepatocellular carcinoma, HBs antigen-positive, synonym of PLC/PRF/5 strain), OCUG-1 (human gallbladder cancer, malignant), huH-1 (human hepatocellular carcinoma, HBs antigen-positive), JHH-2 (human hepatocellular carcinoma), JHH-5 (human hepatocellular carcinoma), JHH-6 (human hepatocellular carcinoma), JHH-7 (human hepatocellular carcinoma, HBV gene is incorporated), OZ (human bile duct carcinoma), NOZ (human gallbladder cancer, moderately differentiated type tubular adenocarcinoma), HepG2 (human hepatocellular carcinoma), JHH-1 (human hepatocellular carcinoma), GS-3A4-HepG2 (human hepatocellular carcinoma, human glutamine synthetase and CYP3A4 forcibly-expressed cell), TYGBK-1 (human gallbladder cancer), TYBDC-1 (human bile duct adenocarcinoma (established from liver metastasis lesion of bile duct carcinoma)), KKU-055 (human bile duct carcinoma cell), MMNK-1 (immortalized cell strain derived from human bile duct), KKU-213 (human bile duct carcinoma cell), KKU-100 (human bile duct carcinoma cell), TWNT-1 (human liver Ito cell, immortalized cell strain of LI90), TYGBK-8 (human gallbladder cancer (moderately differentiated type tubular carcinoma)), and DR-EcoScreen (cell strain used for in vitro transcription activation assay of mouse aryl hydrocarbon receptor).

In a particularly preferred embodiment, artificial hepatocytes that have an ammonia-metabolizing ability of 100 µg/dl/24 h or higher, and can constitute a reticular structure are used. Such highly functional artificial hepatocytes will be explained in detail.

### <Artificial hepatocytes>

### [Reticular structure]

The expression "cells can constitute a reticular structure" used for characteristics of cells means that if the target cells are cultured under appropriate conditions, a predetermined reticular structure is constituted by the cells. Such a reticular structure can be constituted by inoculating cells at an appropriate density into an appropriate medium contained in a culture vessel, which may be coated with an extracellular matrix (ECM), such as Matrigel (registered trademark), hyaluronic acid, heparin, fibronectin, laminin, vitronectin, and other ECMs as required, and culturing the cells for several days. Such a reticular structure contains parts in the shape of string having a width of a size corresponding to at least one cell to 1000 µm (more specifically a size corresponding to several cells to 500 µm, further specifically 20 to 250 µm), and parts of voids having a circular shape, elliptical shape, or the like. The diameter of the voids is typically 100 to 2000 µm, more specifically 200 to 1000 µm, further specifically 300 to 1000 µm. The reticular structure may have a thickness of 10 to 60 µm, which corresponds to the size of 1 to 3 cells.

### [Ammonia-metabolizing ability]

The term ammonia-metabolizing ability used for the present invention means ammonia-metabolizing ability observed when target cells are cultured under appropriate conditions, unless especially indicated. Although methods for measuring ammonia-metabolizing ability are well known to those skilled in the art, adhesion cultivation is preferably performed using the compound mentioned later (FH1) and a culture vessel, which may be coated with ECM as required, without using feeder cells (HUVECs, MSCs, etc.). As for more detailed conditions, the method described in the section of Example in this specification can be referred to.

In a preferred embodiment, the artificial hepatocytes have a high ammonia-metabolizing ability. Specifically, the artificial hepatocytes may have an ammonia-metabolizing ability of 100 µg/dl/24h or higher, preferably 120 µg/dl/24h or higher, more preferably an ammonia-metabolizing ability comparable to the metabolizing ability of the primary hepatocytes (160 to 200 µg/dl/24h), i.e., 160 µg/dl/24h, further preferably 180 µg/dl/24h).

### [Characteristics of culture]

### Culture under serum-free and/or feeder-free environment

As the medium for culturing the artificial hepatocytes, various existing media developed for culturing hepatocytes can be used. Examples of usable medium include the differentiation induction medium III described later, HCM™ BulletKit™ Medium (Lonza Walkersville, Inc), and HBM™ Basal Medium (Lonza Walkersville, Inc).

The artificial hepatocytes can be cultured under a serum-free and/or feeder-free environment, preferably a serum-free and feeder-free environment. The expression of culturing under a serum-free and feeder-free environment means to culture the cells by using a medium not containing animal serum or human serum under an environment where cells other than the target artificial hepatocytes, for example, mouse fetal fibroblasts and feeder cells (human umbilical vein endothelial cells, (HUVECs)) and mesenchymal stem cells (MSCs), do not exist.

### ECM coating

For culturing the artificial hepatocytes, various kinds of existing culture vessels can be used. Although the material of the culture vessel can be appropriately chosen by those skilled in the art, a culture vessel coated with ECM can be preferably used from the viewpoints of obtaining favorable proliferation and maintaining high functions. Preferred examples of ECM include Matrigel (registered trademark), hyaluronic acid, heparin, fibronectin, laminin, vitronectin, proteoglycan (chondroitin sulfate proteoglycan, heparan sulfate proteoglycan, keratan sulfate proteoglycan, and dermatan sulfate proteoglycan), various collagens, gelatin, tenascin, entactin, elastin, fibrillin, and so forth.

### Maintenance culture and subculture

It has been confirmed that the artificial hepatocytes can be continuously cultured for about 10 days by using the differentiation induction medium III mentioned later. It is considered that, by inoculating the cells at an appropriate density, they can be subcultured and proliferated. The appropriate density is, for example, such a density that the artificial hepatocytes exactly touch with one another. A density lower than such a density provides poor proliferation, and a density higher than such a density may induce marked cell death in a region where construction of a three-dimensional structure is observed.

### <Method for producing artificial hepatocytes>

The artificial hepatocytes used in a preferred embodiment can be produced by a method comprising the following steps:
the differentiation induction step 1 of culturing an iPS cell in a differentiation induction medium I containing activin A,
the differentiation induction step 2 of culturing a cell obtained in the differentiation induction step 1 in a differentiation induction medium II containing bone morphogenetic protein 4 (BMP4) and fibroblast growth factor 2 (FGF2); and
the differentiation induction step 3 of culturing a cell obtained in the differentiation induction step 2 in a differentiation induction medium III containing hepatocyte growth factor (HGF), oncostatin M, dexamethasone, and a compound having the N-phenyl-2-(N-phenylmethylsulfonamido)acetamide structure to obtain an artificial hepatocyte.

### Differentiation induction step 1

In the differentiation induction step 1, iPS cells are cultured in the differentiation induction medium I containing at least activin A.

### iPS cells

In order to obtain hepatocytes to be used in a preferred embodiment, iPS cells derived from a human or nonhuman animal are used as starting cells. Methods for obtaining and establishing iPS cells are well known to those skilled in the art. Human iPS cells can be purchased from the Institute of Physical and Chemical Research (Riken) BioResource Research Center, or distributed from Kyoto University or National Center for Child Health and Development. Examples of available preferred iPS cells include, for example, those of the human artificial pluripotent stem (iPS) cell strain HiPS-RIKEN-1A established by introducing the four factors (Oct3/4, Sox2, Klf4, c-Myc) into an umbilical cord-derived fibroblast (RCB0436 HUC-F2) using a retroviral vector (Institute of Physical and Chemical Research, HPS0003), human iPS cell strain HiPS-RIKEN-2A established by introducing the four factors (Oct3/4, Sox2, Klf4, c-Myc) into an umbilical cord-derived fibroblast (RCB0197 HUC-Fm) by using a retroviral vector (Institute of Physical and Chemical Research, HPS0009), human iPS cell strain HiPS-RIKEN-12A obtained by introducing the three factors (Oct3/4, Sox2, Klf4) into an umbilical cord-derived fibroblast by using a retroviral vector (Institute of Physical and Chemical Research, HPS0029), human iPS cell strain Nips-B2 established by introducing the four factors (Oct3/4, Sox2, Klf4, c-Myc) using a Sendai Virus vector (Institute of Physical and Chemical Research, HPS0223), and so forth.

### Use of Sendai virus vector

When an iPS cell strain is established, it is preferable to use a Sendai virus vector as a vector for introducing the factors. Since a retroviral vector enters into the nucleus of cell and expresses a gene as DNA, it is concerned that such a vector may positively enter into a patient's chromosome or cause genetic recombination with a chromosomal DNA when the obtained iPS cell is used for a patient, although such phenomena occur very rarely. However, a Sendai virus vector does not enter into a cell nucleus, and it reproduces its genome within cytoplasm to produce a large amount of a protein. Since this genome is made from RNA, and materially differs from DNA of patient's chromosomes, it is considered that there is theoretically no risk for a Sendai virus vector to modify chromosomes in the cell nuclei of patients.

The method for establishing an iPS cell using a Sendai virus (SeV) vector is well known to those skilled in the art, and it can be established by, for example, culturing a commercially available human fibroblast or the like in a medium containing a Sendai virus vector carrying a reprogramming factor expression unit. Examples of such a Sendai virus vector carrying a reprogramming factor expression unit include, for example, CytoTune-iPS (DNAVEC Corporation), and so forth.

### Differentiation induction medium I

The differentiation induction medium I used in this step contains a cytokine such as Wnt3A or activin A. It preferably contains at least activin A. Amount of the cytokine is, for example, about 10 to 50 ng/mL, preferably about 25 ng/ml, in the case of Wnt3A, or about 10 to 100 ng/ml, preferably about 100 ng/ml, in the case of activin A.

The differentiation induction medium I is serum-free, and for example, a medium obtained by adding such a cytokine as mentioned above and a serum substitute to a basic composition such as RPMI1640 can be used. Examples of such a serum substitute include B27 (registered trademark, Life Technologies), KnockOut (registered trademark) Serum Replacement (Life Technologies), and so forth. An example of specific composition of the differentiation induction medium I is the composition described in the section of Example of this specification. As for the composition of B27, G.J. Brewer et al., Optimized Survival of Hippocampal Neurons in B27-Supplemented Neurobasal™, a New Serum-free Medium Combination, Journal of Neuroscience Research 35567476 (1993) can be referred to.

This step is performed by culturing iPS cells at 37°C for several days in a 5% CO₂ incubator by using the differentiation induction medium I. The culture period of the differentiation induction step 1 is specifically 4 to 10 days, preferably 6 to 8 days, more preferably 7 days. The differentiation induction step 1 can be performed until the cells come to have the morphological characteristics of endomere.

### Differentiation induction step 2

In the differentiation induction step 2, the cells obtained from the differentiation induction step 1 are cultured in the differentiation induction medium II.

### Differentiation induction medium II

The differentiation induction medium II used in this step contains various cytokines, for example, fibroblast growth factor 2 (FGF2), bone morphogenetic protein 4 (BMP4), and hepatocyte growth factor (HGF). The amount of the cytokine is, for example, 5 to 50 ng/ml in the case of FGF2, 5 to 50 ng/ml in the case of BMP4, or 5 to 50 ng/ml in the case of HGF, preferably about 10 ng/ml in the case of FGF2, about 20 ng/ml in the case of BMP4, or about 20 ng/ml in the case of HGF. The differentiation induction medium II contains at least the bone morphogenetic protein 4 (BMP4) and fibroblast growth factor 2 (FGF2). The differentiation induction medium II can also be serum-free, and for example, a medium obtained by adding the aforementioned cytokines and a serum substitute to a fundamental composition such as RPMI1640 can be used. A specific example of the differentiation induction medium II is one having the composition described in the section of Example of this specification.

This step can be carried out by culturing the cells obtained in the step 1 at 37°C in a 5% CO₂ incubator by using the differentiation induction medium II. The culture period of the differentiation induction step 2 is specifically 1 to 6 days, preferably 2 to 5 days, more preferably 3 to 4 days.

### Differentiation induction step 3

This step is a step of culturing the cell obtained from the differentiation induction step 2 in the differentiation induction medium III to obtain artificial hepatocytes.

### Differentiation induction medium III

Examples of the cytokine or hormone used in this medium include oncostatin M (OSM), dexamethasone, and so forth. The amount thereof to be used is, for example, 10 to 50 ng/ml in the case of OSM, or 0.05 to 0.5 µM in the case of dexamethasone, preferably about 25 ng/ml in the case of OSM, or about 0.1 µM in the case of dexamethasone. The differentiation induction medium III contains at least hepatocyte growth factor (HGF), oncostatin M, dexamethasone, and N,N'-(methylenebis)(4,1-phenylene)diacetamide (FH1) and/or 2-(N-(5-chloro-2-methylphenyl)(methylsulfonamido)-N-(2,6-difluorophenyl)acetamide (FPH1). A specific example of the differentiation induction medium III is one having the composition described in the section of Example of this specification.

### FH1 and FPH1

The differentiation induction medium III contains N,N'-(methylenebis)(4,1-phenylene)diacetamide (FH1) and/or 2-(N-(5-chloro-2-methylphenyl)(methylsulfonamido)-N-(2,6-difluorophenyl)acetamide (FPH1) represented by the following formulas.

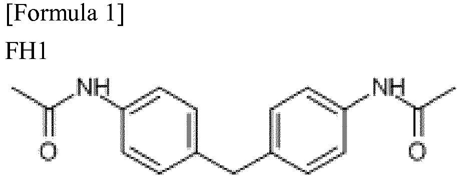

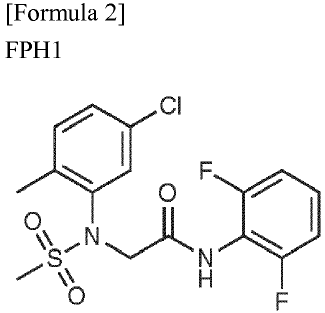

In a preferred embodiment, the differentiation induction medium III contains FH1 and FPH1.

This step can be carried out by culturing the cells obtained in the differentiation induction step 2 in the differentiation induction medium III for several days to several weeks. In this culture period, it is preferable to exchange the medium at a frequency of at least every 3 days, preferably everyday.

### Other conditions

Each of the differentiation induction steps 1 to 3 can be performed under a serum-free and/or feeder-free environment, preferably a serum-free and feeder-free environment. They can also be carried out by using a culture vessel coated with ECM as required.

### Confirmation of hepatocytes

It is known that hepatic parenchymal cells embryologically appear following the appearance of the cardiac muscle cells in the embryoid body. It is experimentally known that expression of hepatic parenchymal cells is observed near cardiac muscle cells differentiated from the embryoid body.

As for the method for confirming the differentiation of the cells obtained after the differentiation induction steps 1 to 3 into hepatocytes, the differentiation can be confirmed on the basis of expression of a gene for a hepatocyte (fetal hepatocyte)-specific marker or expression of a hepatocyte-specific marker protein such as transthyretin (TTR), α-fetoprotein (AFP), fetal hepatocyte, a1-antitrypsin (AAT), tyrosine aminotransferase (TAT), tryptophan oxygenase (TO), tyrosine aminotransferase, asialoglycoprotein receptor (ASGR), and albumin, immunostaining using an anti-albumin antibody, or the like. Since many binuclear cells are observed among mouse hepatocytes, the confirmation can also be performed by confirming morphology of cell nuclei.

The confirmation is preferably performed on the basis of the ability to constitute a reticular structure and high ammonia-metabolizing ability as described in the section of artificial hepatocytes mentioned above.

### Examples

### <Preparation of iPS cells>

### [Preparation of iPS cells]

iPS cells were prepared by the following method.
1. Thaw POIETICS (registered trademark) CORD BLOOD CD34+ Cells (Cat. No. 2C-101A) and POIETICS (registered trademark) Mobilized CD34+ Cells (Cat. No. 2G-101B).
2. Prepare iPS cells by using CytoTune (registered trademark)-IPS 2.0 Sendai Reprogramming Kit according to the protocol attached to the kit.

### [Confirmation of iPS cells]

Acquisition of iPS cells was confirmed by performing the following procedure.

Immunostaining
1. Stick 253G1 (obtained from Institute of Physical and Chemical Research (Riken)) and CiPS to slide glass using CytoSpin (registered trademark) (800 rpm, 5 minutes).
2. Carry out fixation with 4% paraformaldehyde (Nacalai Tesque) (15 minutes, room temperature).
3. Carry out washing 3 times with PBS.
4. Carry out blocking (0.3% Triton X-100, 1% BSA, 10% AB serum, or Blocking One-Histo (Nacalai Tesque)), 10 to 20 minutes, room temperature.
5. Carry out a treatment with 0.1% Tween in PBS, 5 minutes, room temperature.
6. Carry out staining with R&D, Human Pluripotent Stem Cell 3-Color Immunocytochemistry Kit, 10-fold dilution for each, 3 hours, room temperature or o/n, 4°C.
7. Carry out washing with PBS
8. Carry out enclosure with VECTA SHIELD (registered trademark) Hard Set with DAPI.
9. Carry out observation with an all-in-one fluorescence microscope BZ-9000 (KEYENCE).

### Flow cytometry

1. Separate 253G1 (obtained from Institute of Physical and Chemical Research (Riken)) and CiPS by a treatment with Accutase at 37°C for 5 minutes, and then collect them.
2. Carry out staining with SSEA-4 FITC, SSEA-3 PE, and TRA-1-81 PE antibodies (final concentration is 1 µg/ml for each) (4°C, 30 minutes).
3. Carry out measurement with FACSCalibur.
4. Carry out analysis with CellQuest or FlowJo.

### Morphological observation

Morphology was periodically observed with the all-in-one fluorescence microscope BZ-9000 (KEYENCE) during the culture performed according the method described later.

### Confirmation of elimination of SeV vector (CytoTune_iPS2.0)

1. Culture the iPS cells on a 12-well plate.
2. Carry out washing with PBS.
3. Carry out fixation with 1 ml/well of 10% neutral formalin (5 minutes, room temperature).
4. Carry out washing with PBS.
5. Add 500 µl of Anti-Sev antibody (diluted 500 times with 0.1% Triton X-100 in PBS).
6. Allow reaction at 37°C for 1 hour.
7. Carry out washing with PBS.
8. Add 500 µl of Dyelight 550-labeled anti-rabbit IgG-antibody (diluted 500 times with 0.1% Triton X-100 in PBS).
9. Allow reaction at 37°C for 1 hour.
10. Carry out washing with PBS.
11. Carry out observation with the all-in-one fluorescence microscope BZ-9000 (KEYENCE).

### Results

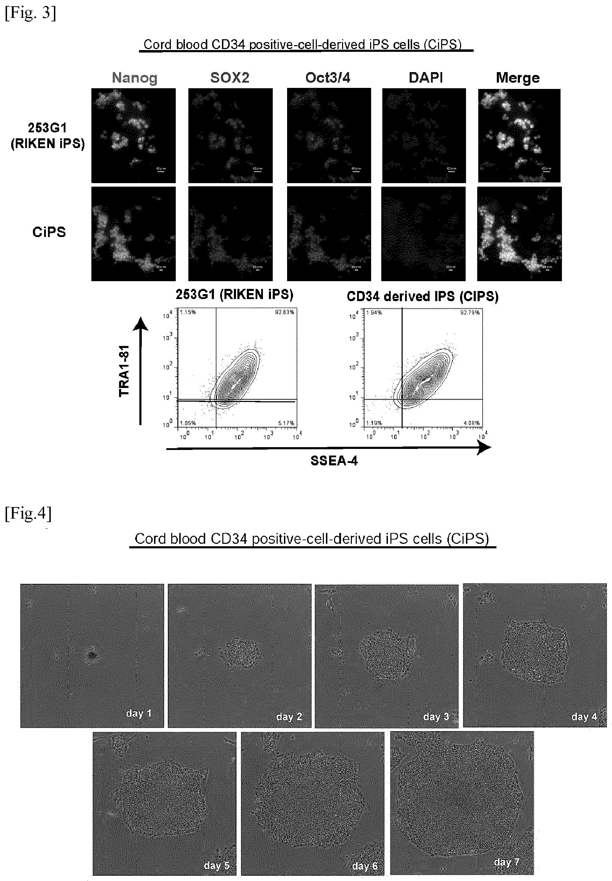

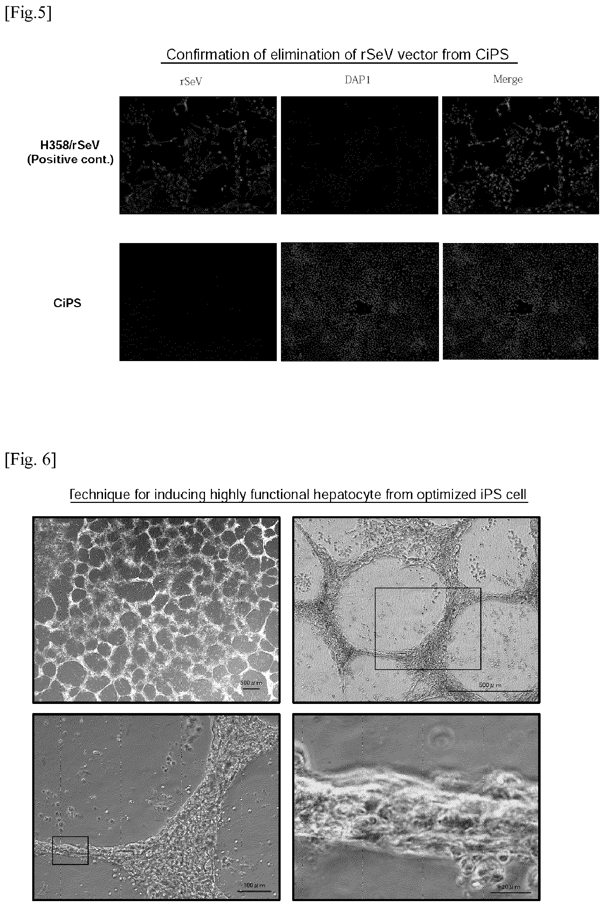

The results of the immunostaining and flow cytometry are shown in Fig. 3, the results of the periodical observation of the cell morphology are shown in Fig. 4, and the results of the confirmation of elimination of the rSeV vector from the CiPS cells are shown in Fig. 5. The CiPS cells were in a feeder-free environment throughout the whole process from the preparation thereof to the expansion culture. CiPS from which sReV had been eliminated could be obtained by subculture for 3 months from the infection.

### <Preparation of iPS cells for inducing CiPS-Hep>

According to the following method, iPS cells for inducing iPS-derived highly functional hepatocytes (CiPS-Hep) were prepared. In the confirmation of the aforementioned prepared iPS cells such as iPS cell colony observation, the methods described in this section were performed.

### [Preparation of laminin-coated dish]

1. Prepare thawed laminin-5¹⁾ (-80°C) at 4°C. Pay attention so as not to warm it with hand.
2. Put PBS³⁾ on a 6-well plate²⁾ in a volume of 1 ml/well, and spread it over each whole well.
3. Cool the 6-well plate containing PBS and 200-µL tip at 4°C.
4. Put the thawed laminin-5 into the wells containing PBS in spiral motion, and quickly sway the plate about 10 times to spread laminin-5 over the whole wells. Since laminin-5 is strongly adsorptive, never perform pipetting.
5. Carry out incubation at 4°C overnight or at 37°C for 2 hours or longer to attain coating. Don't leave it at 37°C.
6. When the plate is not used immediately, it can be doubly sealed with Parafilm and stored at 4°C (1 week)

### [Thawing and culture of frozen cells]

1. Set a P2 centrifugal separator at 20°C.
2. Take out various inhibitors (4 kinds to be used in the step 9 mentioned below) from a freezer at -30°C.
3. Prepare a culture medium (henceforth referred to as ReproFF2).

**[Table 1]**

| | | | |
|---|---|---|---|
| ReproFF2⁴⁾ | | 500ml | |
| + FGF2 ⁵⁾ | lmg/ml | 2.5µl | (Final 5ng/ml) |
| + penicillin/ streptomycin⁶⁾ | | 5ml | (Final 1%) |

4. Put 9 ml or 4 ml each of ReproFF2 into 15-ml tubes⁷⁾, and warm them at 37°C.
5. Rinse the laminin-coated dish twice with PBS(-), and put 1 ml each of PBS(-) into the wells.
6. Take out frozen cells from liquid nitrogen, and thaw them by immersion into a water bath at 37°C for 2 minutes. When the volume of the frozen cells is small, add an appropriate volume of warmed medium, and perform gentle pipetting to quickly thaw them.
7. Collect the thawed cells and move them into a 15-ml tube containing 9 ml of the culture medium, and gently invert the tube to mix them.
8. Centrifuge them under the conditions of 160 g, 5 minutes, and room temperature with acceleration and braking.
9. Separately prepare 4 ml of culture medium containing each inhibitor during the centrifugation.

**[Table 2]**

| | | | |
|---|---|---|---|
| ReproFF2 | | | 4ml |
| + Thiazovivin⁸⁾ | 5mM | 4µl | (Final 5 µM) |
| + CHIR99021⁹⁾ | 1mM | 4µl | (Final 1 µM) |
| +PD0325901¹⁰⁾ | 400µM | 4µl | (Final 0.4µM) |
| + SB431542¹¹⁾ | 2mM | 4µl | (Final 2µM) |

10. Collect the 15-ml tubes after centrifugation, and carefully remove the supernatant.
11. Add 1 ml of the culture medium containing an inhibitor, and gently loosen the cell pellet by pipetting.
12. Remove PBS(-) on the laminin-coated dish and move the culture medium suspending the cells to the wells. Wash the 15-ml tube with the remaining culture medium containing an inhibitor, and move the medium to the well.
13. Sway the dish to uniformly spread the cells and carry out incubation at 37°C under 5% CO₂.
14. On the next day, gently mix the medium contained in the wells, and then remove the supernatant.
15. Add 4 ml of the culture medium containing one of the various kinds of inhibitors again, and culture the cells at 37°C and 5% CO₂.

### [Medium exchange]

1. Remove the culture supernatant.
2. Add fresh culture medium ReproFF2 in a volume of 4 ml/well.
3. Carry out the aforementioned operation every two to three days (on Monday, Wednesday, and Friday).

### [Subculure of cells]

1. Prepare a laminin-coated dish.
2. Remove the culture supernatant.
3. Add 1 ml of PBS(-) to wash cell surfaces.
4. Remove PBS(-), add ESGRO Complete Accutase¹²⁾ in a volume of 1 ml/well, and carry out incubation at 37°C and 5% CO₂ for 5 minutes.
5. Separate the cells not completely separated in the wells by pipetting, and collect the cell suspension into a 15-ml tube.
6. Add 1 ml of ReproFF2 to the well to wash the inside of the well, collect ReproFF2 into a 15-ml tube, and further add 1 ml of ReproFF2 into the 15-ml tube to obtain a total volume of 3 ml.
7. Sufficiently mix the prepared cell suspension, dilute it twice with a trypan blue staining solution¹³⁾, and count the cells.
8. Put the cell suspension into new 15-ml tubes so that subculture can be performed at a density of 5 x 10⁵ cells/well in the wells in which the subculture is performed. Cryopreserve the remaining cells as required.
9. Carry out centrifugation under the conditions of 160 g, 5 minutes, and room temperature with acceleration and braking.
10. Prepare 4 ml/well of the culture medium containing each inhibitor during the centrifugation.

**[Table 3]**

| | | | |
|---|---|---|---|
| ReproFF2 | | | 4ml |
| + Thiazovivin | 5mM | 4µl | (Final 5µM) |

11. After the centrifugation, remove the supernatant, loosen the cell pellet by pipetting with the culture medium containing an inhibitor, and inoculate the cells into each laminin-coated well.
12. Indicate date, name of iPS cells, and number of times of subculture on the culture plate, and carry out culture at 37°C and 5% CO₂. Carry out the subculture operation on Monday, Friday, Wednesday, and Monday again to continue the subculture.

### [Cryopreservation of cells]

1. Separate the cell in the same manner as that used at the time of subculture, and count them.
2. Centrifuge the cell suspension under the conditions of 160 g, 5 minutes, and room temperature with acceleration and braking.
3. Label date, cell name, and number of times of subculture on serum tubes¹⁴⁾ used for cryopreservation.
4. Sufficiently remove the supernatant after centrifugation, and suspend the cells in STEM-CELLBANKER 15 at a density of 1 x 10⁶ cells/500 µl.
5. Put the cell suspension into the labeled serum tubes in a volume of 500 µl each.
6. After the addition of the cell suspension, put the serum tubes into a deep freezer at - 80°C.
7. Transfer the cells frozen at -80°C to a liquid nitrogen cell preservation system within 1 to 3 days, and store them.

### <Induction of CiPS-Hep>

By the following method, differentiation of CiPS cells into highly functional hepatocytes (CiPS-Hep) was induced.

[Day 1: Step 1 for induction of differentiation into iPS-derived highly functional hepatocyte, medium change with differentiation induction medium I for inducing iPS-derived highly functional hepatocyte (henceforth referred to as medium I)]
1. Prepare the medium I in a required volume (4 ml/well).

**[Table 4]**

| | | |
|---|---|---|
| Serum-free RPMI 1640¹⁶⁾ | | 4ml |
| + B27¹⁷⁾ 50x (Final 1x) | | 80µl |
| + recombinant human Active A ¹⁸⁾ | 10µg/ml (Final 100ng/ml) | 40µl |

2. Remove the supernatant, and add 1 ml of serum-free RPMI1640 to wash cell surfaces.
3. Add the medium in a volume of 4 ml/well.
4. Carry out an operation of removing the supernatant and adding fresh medium I every day up to day 6.

[Day 7: Step 2 for inducing differentiation into iPS-derived highly functional hepatocyte; medium change with differentiation induction medium II for inducing iPS-derived highly functional hepatocyte (henceforth referred to as medium II)
1. Prepare the medium II in a required volume (4 ml/well).

**[Table 5]**

| | |
|---|---|
| Serum-free RPMI 1640 | 4ml |
| + B27 50x (Final 1x) | 80µl |
| + BMP4¹⁹⁾ 10µg/ml (Final 20ng/ml) | 8µl |
| + FGF2 10µg/ml (Final 10ng/ml) | 4µl |

2. Carry out gentle pipetting of the culture supernatant, then remove the supernatant, and add the medium II in a volume of 4 ml/well.
3. Carry out an operation of removing the supernatant and adding fresh medium II every day up to day 9.

[Day 10: Step 3 for inducing differentiation into iPS-derived highly functional hepatocyte]

### 1. Coating of culture plate with Matrigel

1.1. Thaw 300 µl of BD Matrigel Growth Factor Reduced²⁰⁾ at 4°C.
1.2. Cool a 24 well-plate dish²¹⁾ and KnockOut-DMEM²²⁾ at 4°C.
1.3. Add an equal volume of KnockOut-DMEM to the thawed Matrigel, and sufficiently mix them by pipetting.
1.4. Put the diluted Matrigel solution into the wells of the 24 well-plate dish in a volume of 200 µl/well, and spread it over the whole well.
1.5. Leave the 24-well plate dish standing at an ordinary temperature for 3 hours or longer to attain coating.

### 2. Subculture of iPS cell-derived highly functional hepatocytes

2.1. Prepare differentiation induction medium III for inducing iPS-derived highly functional hepatocyte (henceforth referred to as medium III) in a volume of 1 ml/well.

**[Table 6]**

| | | |
|---|---|---|
| HCM²³⁾(HBM²⁴⁾ + cocktail) | | 1ml |
| + HGF²⁵⁾ 10µg/ml | (Final 20ng/ml) | 2µl |
| + Oncostatin M²⁶⁾ | 10µg/ml (Final 10ng/ml) | 1µl |
| +Dexamethasone²⁷⁾ | 0.1mM (Final 0.1µM) | 1µl |
| +FH1²⁸⁾⁻¹ | 25mM (Final 50µM) | 2µl |
| | | |
| HCM²³⁾ (HBM²⁴⁾ + cocktail) | | 1ml |
| + HGF²⁵⁾ 10µg/ml | (Final 20ng/ml) | 2µl |
| + Oncostatin M²⁶⁾ | 10µg/ml (Final 10ng/ml) | 1µl |
| + Dexamethasone²⁷⁾ | 0.1mM (Final 0.1µM) | 1µl |
| + FH1²⁸⁾⁻¹ | 25mM (Final 50µM) | 2µl |
| + FPH1²⁸⁾⁻² | 12.86mM(Final 20µM) | 1.56µl |

2.2. Remove the culture supernatant and wash cell surfaces with 1 ml of PBS(-).
2.3. Add 1 ml of ESGRO Complete Accutase, and carry out incubation at 37°C for 5 minutes.
2.4. Carefully carry out pipetting to separate the cells and collect the cell suspension into a 15-ml tube.
2.5. Wash the inside of the well with 1 ml of HCM, collect the solution into the 15-ml tube, and further add 1 ml of HCM into the 15-ml tube to obtain a total volume of 3 ml. 2.6. Sufficiently mix the cell suspension, and count the cells with 2-fold dilution of trypan blue.
2.7. Put the cell suspension into new 15-ml tubes so that subculture can be performed at a density of 1.0 x 10⁶ cells/well in the wells in which the subculture is performed.
2.8. Carry out centrifugation at 160 g and room temperature for 5 minutes.
2.9. During the centrifugation, add 1 ml of PBS(-) to the Matrigel-coated well for washing, and remove the supernatant. Repeat this washing operation twice, and add 1 ml of PBS(-) to the well.
2.10. After the centrifugation, remove the supernatant. When adhesion cultivation is performed, remove also the supernatant of the Matrigel-coated well, loosen the cell pellet by pipetting using the medium III, and inoculate the cells to the Matrigel-coated well.

When floating cultivation is performed, loosen the cell pellet by pipetting using the medium III, and inoculate the cells to MPC treatment plate (MD6 with Lid Low-Cell Binding, Nalge Nunc International, Japan).

When HUVEC and MSC are used as feeder cells, perform co-culture as adhesion cultivation as required (refer to Non-patent document 1 mentioned below). 2.11. Indicate date, name of cell etc. on the culture plate, and perform the cultivation at 37°C in a 5% CO₂ incubator.

2.12. Carry out an operation of removing the supernatant and adding fresh medium III every other day up to day 17.

[Day 17: Completion of CiPS-Hep -> ammonia metabolism test]
1. Prepare HBM (cocktail) + aqueous ammonia (henceforth referred to as ammonia medium) in a volume of 1 ml/well x 2.

**[Table 7]**

| | |
|---|---|
| HBM | 1 ml |
| Aqueous ammonia²⁹⁾ (1 M) | 2 µl (2mM) |

2. Remove the culture supernatant, and wash the cell surfaces once with 1 ml of HBM and once with ammonia HBM.
3. Remove the supernatant, and add the ammonia medium in a volume of 1 ml/well.
4. Put 1 ml each of the ammonia medium and HBM into empty wells for positive and negative controls.
5. Carry out cultivation at 37°C and 5% CO₂ for 24 hours.

[Day 18: Collection of samples for ammonia metabolism test]
1. Collect the supernatant into an Eppendorf tube and subjected it to centrifugation at 500 g for 5 minutes.
2. Collect the supernatant into three new Eppendorf tubes in a volume of 300 µl each, and freeze it at -80°C.
3. Wash each well with PBS, add 1 ml of trypsin EDTA³⁰⁾, and carry out incubation at 37°C for 5 minutes.
4. Carefully carry out pipetting to separate the cells and pass the cell suspension through an FACS tube³¹⁾.
5. Carry out washing with 1 ml of HBM, and make the total volume 2 ml.
6. Count the cells with trypan blue.

### <Confirmation of iPS-HEP cells>

The following procedures were performed to confirm that iPS cells had been obtained.

### Ammonia Test Wako³²⁾

1. Thaw the supernatant.
2. Carry out centrifugation at 8000 rpm and 4°C for 5 minutes.
3. Prepare an ammonia dilution. Not use the standard solution and standard dilution contained in the kit. Add 40 µl of the ammonium ion standard solution (1000 mg/L) to 960 µl of HBM (additive-free), and mix them with a vortex mixer to obtain a standard solution.

**[Table 8]**

| |
|---|
| 0 µg/dl: HBM alone |
| 100 µg/dl: Standard solution (100 µl) + HBM (300 µl) |
| 200 µg/dl: Standard solution (150 µl) + HBM (150 µl) |
| 300 µg/dl: Standard solution (300 µl) + HBM (100 µl) |
| 400 µg/dl: Standard solution alone |

4. Transfer the supernatant of the sample and the ammonia dilution in a volume of 200 µl each to a new Eppendorf tube.
5. Add 800 µl of a deproteinization solution, and mix them with a vortex mixer.
6. Carry out centrifugation at 8000 rpm and 4°C for 5 minutes.
7. During the centrifugation, turn on electric sources of a microplate reader and personal computer, and select the Tecan i-control mode.
8. Put the supernatant into wells of a 96-well flat bottom plate in a volume of 60 µl each in duplicate.
9. Add a color developing test solution A to the wells in a volume of 60 µl each.
10. Remove the lid of the microplate reader, set the plate on the microplate reader, and shake the plate (Double click "Shaking", set the time 10 seconds, select "Normal", and click "Stat").
11. Add a color developing test solution B to the wells in a volume of 30 µl each.
12. Set the plate on the microplate reader, and shake the plate.
13. Add a color developing test solution C to the wells in a volume of 60 µl each as quickly as possible.
14. Set the plate on the microplate reader, and shake the plate.
15. Carry out incubation at 37°C for 20 minutes in a 5% CO₂ incubator.
16. Terminate the reaction by cooling at 4°C for 10 minutes or longer.
17. Finely wipe the bottom of the plate, and set the place on the microplate reader by fitting the upper left corner thereof.
18. Measure absorbance (620 or 630 nm) (Double click "Absorbance" of "Measure", select wells for which the measurement is performed in the "Details" mode at 620 nm, and click "Start").

### RT-PCR

1. Carry out RNA extraction by using ISOGEN II (NIPPON GENE) according to the attached protocol.
2. Measure RNA concentration by using NanoDrop (registered trademark).
3. Synthesize cDNA by using PrimeScript High Fidelity RT-PCR Kit.
4. Carry out PCR by using Veriti 96-Well Thermal Cycler (Applied Biosystems).
5. The primer sets are as follows.

**[Table 9]**

| Primer Name | Sequence | SEQ ID NO.: |
|---|---|---|
| hALB For | CCTTTGGCACAATGAAGTGGGTAACC | 1 |
| hALB Rev | CAGCAGTCAGCCATTTCACCATAGG | 2 |
| hHNF4a For | CTGCTCGGAGCCACCAAGAGATCCATG | 3 |
| hHNF4a Rev | ATCATCTGCCACGTGATGCTCTGCA | 4 |
| hOCT4 For | GACAACAATGAAAATCTTCAGGAGA | 5 |
| hOCT4 Rev | TTCTGGCGCCGGTTACAGAACCA | 6 |
| hCYP3A4 For | CCTTACATATACACACCCTTTG | 7 |
| hCYP3A4 Rev | GGTTGAAGAAGTCCTCCTAAGCT | 8 |
| hβ-actin For | TCACCACCACGGCCGAGCG | 9 |
| hβ-actin Rev | TCTCCTTCTGCATCCTGTCG | 10 |

7. Carry out electrophoresis of the RT-PCR products on 1% agarose gel.
8. Stain the gel with Gel Red and observe it.

### Results

The photographs of the obtained iPS-HEP cells are shown in Fig. 6. The obtained cells constituted a characteristic reticular structure. The diameters of the voids were about 300 to 1000 µm, and the widths of the net portions were about 20 to 200 µm. The structure has a thickness of about 10 to 60 µm corresponding to the width of 1 to 3 cells.

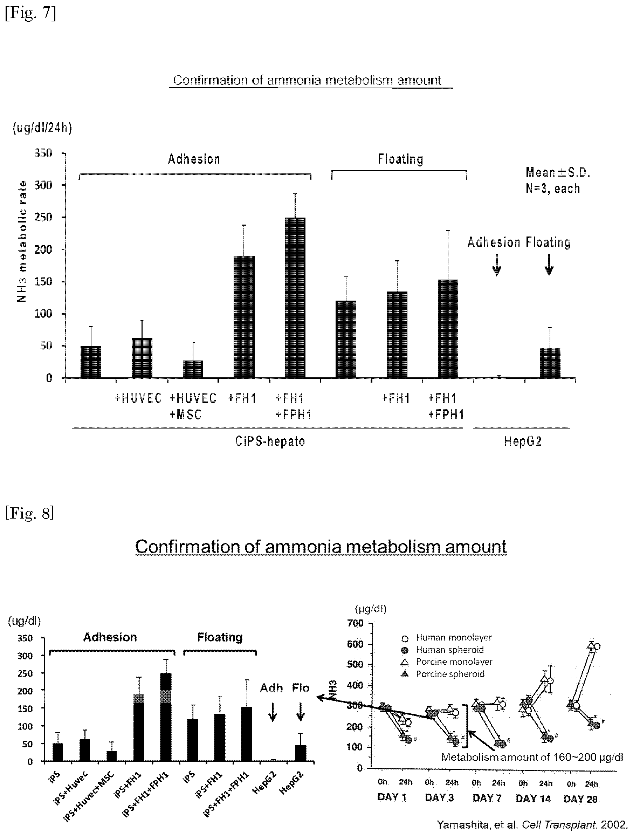

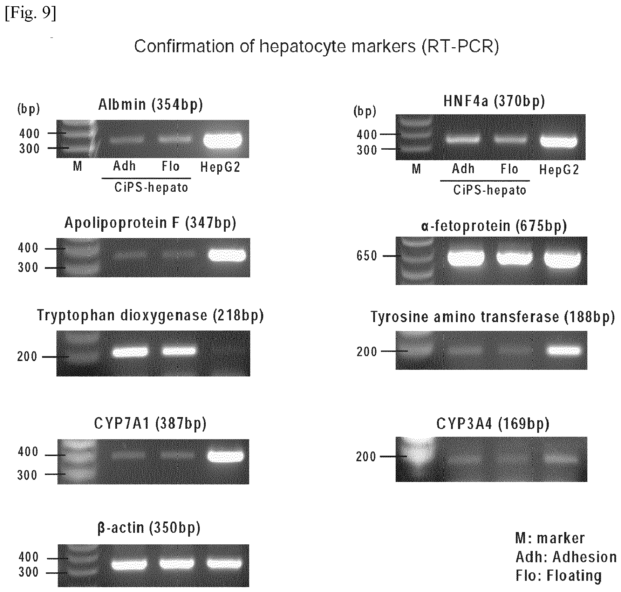

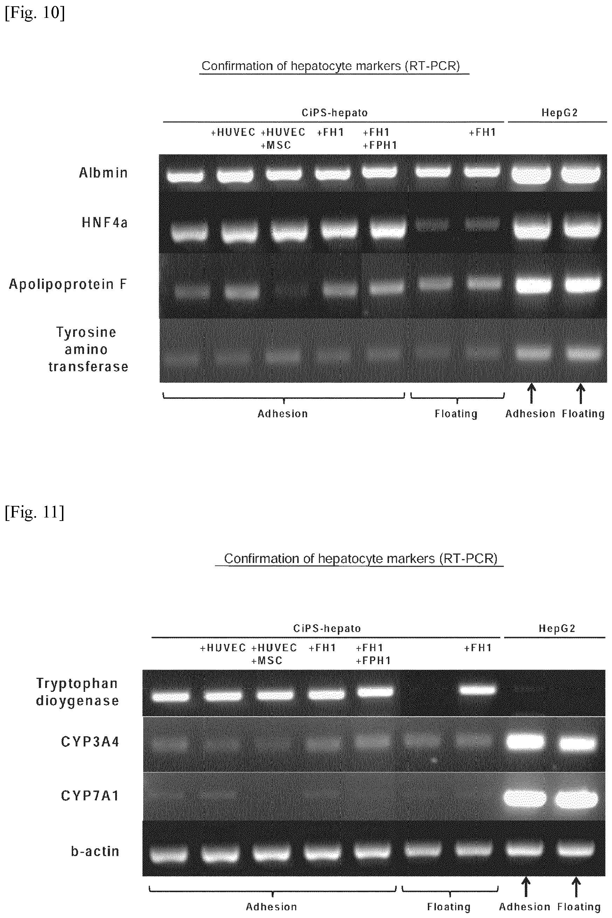

The obtained results of the ammonia metabolism test and RT-PCR are shown in Figs. 7 to 11. It was found that the iPS-HEP cells can have an ammonia-metabolizing ability comparable to the metabolizing ability of the primary hepatocytes (160 to 200 µg/dl/24h) depending on the culture conditions. Further, higher ammonia-metabolizing ability-enhancing effect was obtained by use of the compounds (FH1 and FPH1) rather than use of feeder cells (human umbilical vein endothelial cells (HUVECs) and mesenchymal stem cells (MSCs)).

### <List of materials used in the example>

**[Table 10]**

| | Reagent | Volume | Manufacturer | Product No. | Storage temperature | |
|---|---|---|---|---|---|---|
| 01) | Laminin - 5 | 1 µg/vial (47 µl) | Repro CELL | RCHE OT004 | -80°C | Thaw on ice. |
| 02) | 6-Well plate | | NUNC | 140675 | | |
| 03) | D-PBS(-) | 500 ml | Wako Pure Chemical Industries | 045-29795 | Room temperature | Use as it is. |
| 04) | ReproFF2 | 500 ml | Repro CELL | RCHE MD006 | -30°C | Store at 4°C after thawing, use within 1 month. |
| 05) | FGF-2 (=BFGF) | 25µg (1mg/ml) | Repro CELL | RCHE OT002 | -80°C | Divide into 5 µl portions, freeze again, store at 4°C after thawing, use within 1 month. |
| 06) | Penicillin-streptomycin mixed solution | 100 ml | Nacalai Tesque | 26253-84 | -30°C | Divide into 5 ml portions. |
| 07) | 15-ml Tube | | NUNC | 339650 | | |
| 08) | Thiazovivin | 5 mg | StemRD | Thia-05 | -30°C | Dissolve with 3.215 ml of DMSO, put into light-shielded tubes in a volume of 260 µl each (5 mM). |
| 09) | Stemolecule™ CHIR99021 in Solution | | STEMGENT | 04-0004-02 | -30°C | Store in light-shielded tube, freeze after every use. |
| 10) | Stemolecule™ PD0325901 in Solution | | STEMGENT | 04-0006-02 | -30°C | Store in light-shielded tube, freeze after every use. |
| 11) | SB431542, 5 mg 10 mM solution in DMSO | | Cellagen Technology | C7243-5s | -30°C | Stored in light-shielded tube, freeze after every use. |
| 12) | Accutase™ | 100 ml | Nacalai Tesque | 12679-54 | -30°C | Store at 4°C after thawing. |
| 13) | 0.4% Trypan blue staining solution | 100 ml | Life Technologie s, Inc | 15250-061 | Room temperature | Use as it is. |
| 14) | Serum tube | | Sumitomo Bakelite | MS-4501X | | |
| 15) | STEM-CELLBANKER | 20 ml | Takara Bio | CB043 | -30°C | Store at 4°C after thawing. |
| 16) | RPMI1640 | 500 ml | Wako Pure Chemical Industries | 189-02025 | 4°C | Use as it is. |
| 17) | B27 Serum-Free Supplement (50x), Liquid | 10 ml | Life Technologie s, Inc | 7504-044 | -30°C | Thaw at 4°C with light shielding, put into light-shielded tubes in a volume of 1 ml each. |
| 18) | Recombinant Human/Murine/ Rat Activin A | 10 µg | Peprotech, Inc | AF-120-14E | -30°C | Dissolve with 1 ml of 0.1% BSA-PBS, divide into 200 µl portions (10 µg/ml). |
| 19) | Stemfactor BMP-4 (Human Recombinant) | 10 µg | Miltenyi Biotec | 130-095-549 | -30°C | HCL? |
| 20) | BD Matrigel Matrix Growth Factor Reduced | 10 ml | BD Biosciences | 3542-30 | -30°C | Thaw on ice, divide into 100 µl or 300 µl portions. |
| 21) | 24 Well-plate dish | | NUNC | 126667 | | |
| 22) | Cell Therapy Systems KnockOut™ DMEM CTS™ (1x) | 500 ml | Life Technologie s, Inc | A12861 -01 | 4°C | Use as it is. |
| 23) | Medium kit for exclusive use for hepatocytes (HCM BulletKit) | | Takara Bio | CC-3199 | 4°C | Store at -30°C as cocktail, store at 4°C after addition. |
| 24) | Medium kit for exclusive use for hepatocytes (HCM BulletKit) | | | CC-3198 | 4°C | |
| 25) | Recombinant Human HGF | 10 µg | Peprotech, Inc | 100-39 | -30°C | Dissolve with 1 ml of 0.1% BSA in PBS, divide into 50 µl portions (10 µg/ml). |
| 26) | Recombinant Human Oncostatin M (227 a.a) | 10 µg | Peprotech, Inc | 300-10 | -30°C | Dissolve with 1 ml of 0.1% BSA in PBS, divide into 50 µl portions (10 µg/ml). |
| 27) | Dexamethasone | | TOCRIS | 1126 | -30°C | Dilute 10 mM solution 10 times with ethanol. |
| 28) -1 | FH1 | 5 mg | Sigma | SML08 26 | 4°C | Dissolve with 708 µl of DMSO, put into light-shielding tube (25 mM). |
| 28) -2 | FPH1 | 5mg | Glixx Laboratories | GLXC-03609 | 4°C | Dissolve with 1 ml of DMSO, put into light-shielding tube (12.86 mM). |
| 29) | Ammonium ion standard solution | 25 ml | Wako | 019-15461 | 25°C or lower | Shield from light. |
| 30) | Trypsin EDTA | 100 ml | Life Technologies , Invitrogen | 25200-056 | 4°C | |
| 31) | FACS tube | | BD FALCON | 352235 | | |
| 32) | Ammonia Test Wako | | Wako | 277-14401 | 4°C | |

### <Reference cited in example>

Non-patent document 1: T. Takebe et al., Vascularized and functional human liver from an iPSC-derived organ bud transplant, Nature, 499, 481-484 (2013), doi:10.1038-/nature 12271, Published online 03 July 2013

### Industrial Applicability

The extracorporeal artificial liver obtained according to the present invention is expected to be used in order to assist functions of the liver in a period until liver transplantation is carried out, or patient's own liver regenerates.

### Description of Notations

1 Plasma ingredient inlet port
2 Plasma ingredient outlet port
3 Plasma ingredient circulation chamber
4 Hepatocyte culture chamber
5 Separation membrane
6 Separation membrane support (means for suppressing deformation of separation membrane)
7 Module lid
8 Body of module
9 Hepatocytes
10 Patient
11 Plasma separator
12 Artificial liver
20 Circulation circuit 1 (circuit on the patient side)
21 Circulation Circuit 2 (circuit on the artificial liver side)

### Sequence Listing Free Text

SEQ ID NO: 1, hALB For
SEQ ID NO: 2, hALB Rev
SEQ ID NO: 3, hHNF4a For
SEQ ID NO: 4, hHNF4a Rev
SEQ ID NO: 5, hOCT4 For
SEQ ID NO: 6, hOCT4 Rev
SEQ ID NO: 7, hCYP3A4 For
SEQ ID NO: 8, hCYP3A4 Rev
SEQ ID NO: 9, hβ-actin For
SEQ ID NO: 10, hβ-actin Rev

## Claims

1. An extracorporeal artificial liver having at least one module comprising the followings:
- a plasma ingredient inlet port, and a plasma ingredient outlet port;
- a plasma ingredient circulation chamber having the plasma ingredient inlet port, and the plasma ingredient outlet port; and
- a hepatocyte culture chamber provided adjacently to the plasma ingredient circulation chamber and separated from the plasma ingredient circulation chamber with a separation membrane through which ammonia can permeate, but hepatocytes cannot permeate, in which hepatocytes having an ammonia-metabolizing ability are cultured.

2. The artificial liver according to claim 1, wherein the plasma ingredient circulation chamber is disposed above the hepatocyte culture chamber.

3. The artificial liver according to claim 1 or 2, wherein the hepatocytes are artificial hepatocytes that have an ammonia-metabolizing ability of 100 µg/dl/24h or higher, and can constitute a reticular structure.

4. The artificial liver according to any one of claims 1 to 3, wherein the hepatocytes are cultured under a serum-free and feeder-free environment.

5. The artificial liver according to any one of claims 1 to 4, wherein at least 1.0 x 10⁶ of hepatocytes are cultured per one module.

6. The artificial liver according to any one of claims 1 to 5, wherein the artificial liver comprises a plurality of modules, and 5.0 x 10⁹ to 5.0 x 10¹¹ of hepatocytes are cultured.

7. The artificial liver according to any one of claims 1 to 6, which has an effective culture area of 1 x 10⁴ to 1 x 10⁶ cm².

8. A device for an extracorporeal artificial liver or for culturing hepatocytes, which comprises at least the followings:
- a plasma ingredient inlet port, and a plasma ingredient outlet port;
- a plasma ingredient circulation chamber having the plasma ingredient inlet port, and the plasma ingredient outlet port; and
- a hepatocyte culture chamber for culturing hepatocytes provided adjacently to the plasma ingredient circulation chamber and separated from the plasma ingredient circulation chamber with a separation membrane through which ammonia can permeate, but hepatocytes and cannot permeate.

9. The device according to claim 8, wherein the hepatocyte culture chamber has a cell introduction port for introducing hepatocytes.

10. The device according to claim 9, wherein the hepatocyte culture chamber has an air-discharging port for discharging air.

11. The device according to any one of claims 8 to 10, wherein a means for suppressing deformation of the separation membrane is provided in the hepatocyte culture chamber.
